# EUROPEAN PATENT SPECIFICATION

(11) **EP 0 385 721 B1**
(45) Date of publication and mention of the grant of the patent: **25.01.1995**
(21) Application number: 90302075.8
(22) Date of filing: 27.02.1990
(51) Int. Cl.: C07D 471/04, C07D 487/04

(54) **Process for the preparation of lactam derivatives**
Verfahren zur Herstellung von Lactamderivaten
Procédé pour la préparation de dérivés de lactames

(30) Priority: 28.02.1989 GB 8904552
(43) Date of publication of application: 05.09.1990
(73) Proprietor: GLAXO GROUP LIMITED, Greenford, Middlesex UB6 ONN (GB)
(72) Inventor: Coates, Ian Harold, Ware, Hertfordshire, SD12 03G (GB); Oxford, Alexander William, Ware, Hertfordshire, SD12 03G (GB); North, Peter, Charles, Ware, Hertfordshire, SD12 03G (GB); Miller, Thomas, Ware, Hertfordshire, SD12 03G (GB); Baxter, Anthony David, Greenford, Middlesex, UB6 0HE (GB); Hammond, Kevin Ian, Ulverston, Cumbria (GB)
(74) Representative: Marchant, James Ian

(56) References cited:
- EP-A- 0 306 323
- EP-A- 0 353 983
- DE-A- 3 740 352

## Description

This invention relates to a process for the preparation of heterocyclic compounds.

In British patent application no. 2209335A, which was unpublished at the priority date of the present application, a group of lactam derivatives are described which may be represented by the general formula (I): 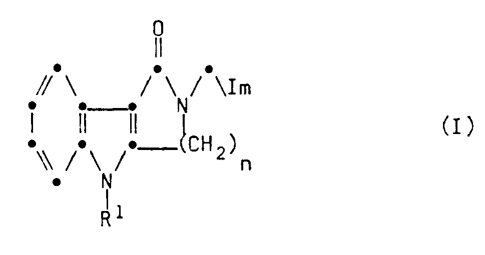
wherein Im represents an imidazolyl group of the formula : 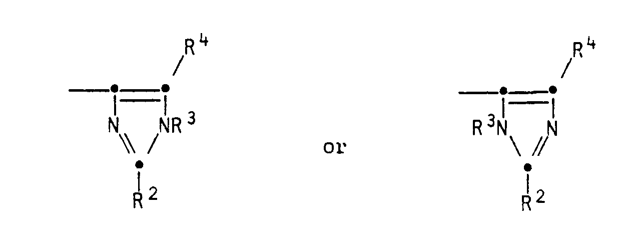
and R¹ represents a hydrogen atom or a group selected from C₁₋₆alkyl, C₃₋₆alkenyl, C₃₋₁₀alkynyl, C₃₋₇cycloalkyl, C₃₋₇cycloalkylC₁₋₄alkyl, phenyl, phenylC₁₋₃alkyl, phenylmethoxymethyl, phenoxyethyl, phenoxymethyl, -CO₂R⁵, -COR⁵, -CONR⁵R⁶ or -SO₂R⁵ (wherein R⁵ and R⁶, which may be the same or different, each represents a hydrogen atom, a C₁₋₆ alkyl or C₃₋₇cycloalkyl group, or a phenyl or phenylC₁₋₄alkyl group, in which the phenyl group is optionally substituted by one or more C₁₋₄alkyl, C₁₋₄alkoxy or hydroxy groups or halogen atoms, with the proviso that R⁵ does not represent a hydrogen atom when R¹ represents a group -CO₂R⁵ or -SO₂R⁵);
one of the groups represented by R², R³ and R⁴ is a hydrogen atom or a C₁₋₆alkyl, C₃₋₇cycloalkyl, C₃₋₆alkenyl, phenyl or phenylC₁₋₃alkyl group, and each of the other two groups, which may be the same or different, represents a hydrogen atom or a C₁₋₆alkyl group;
n represents 2 or 3;
and physiologically acceptable salts and solvates thereof.

Several processes for the preparation of these compounds are described in the abovementioned British patent application.

As described in British patent application no. 2209335A, the compounds of formula (I) are potent and selective antagonists of 5-hydroxytryptamine (5-HT) at 5-HT₃ receptors. They are useful in the treatment of conditions such as psychotic disorders (e.g. schizophrenia and mania); anxiety; and nausea and vomiting, particularly that associated with cancer chemotherapy and radiotherapy.

In EP-A-353 983, which was published after the priority date of the present application, the preparation of tricyclic lactams of the general formula (I) 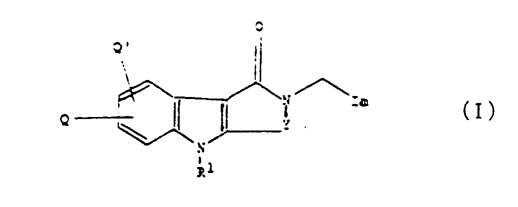
wherein Im, R¹ have the above meanings,
a' represents a hydrogen or a fluorine atom,
Y represents the group CH=CH or (CH₂)ₙ, wherein n represents 2 or 3;
Q represents a halogen atom, or a group selected from hydroxy, C₁₋₄alkoxy, phenylC₁₋₃alkoxy-, C₁₋₆alkyl, cyano, phenyl which may be unsubstituted or substituted,
by alkylating a compound of formula (II) 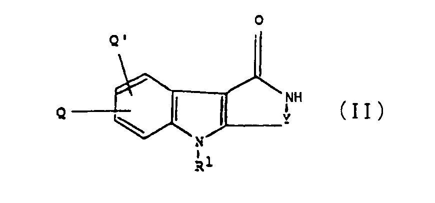
with HOCH₂-Im in the presence of an acid at an elevated temperature, is disclosed.

The present invention provides a process for the preparation of a compound of general formula (I) which comprises reacting a compound of formula (II): 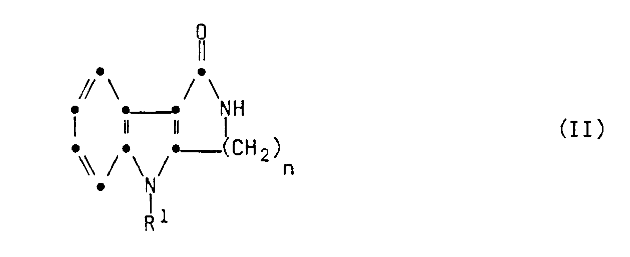
or a protected derivative thereof, with a compound of formula (III):

HOCH₂-Im (III)

or a salt thereof, in the presence of an acid at an elevated temperature, followed where necessary by removal of any protecting groups.

The acid may be, for example, a strong mineral acid (e.g. hydrochloric acid), a hydrocarbylsulphonic acid (e.g. p-toluenesulphonic or methanesulphonic acid), or a carboxylic acid (e.g. maleic or acetic acid).

The reaction may conveniently be effected in a high boiling polar solvent such as N-methylpyrrolidinone or dimethylacetamide, at an elevated temperature, for example in the range 100 to 200°C. Alternatively the reaction may be conveniently effected in water, an alcohol (e.g. isopropanol or n-butanol), xylene or acetic acid at the reflux temperature of the solvent.

According to one aspect of the invention, the acid may be, for example, a strong mineral acid (e.g. hydrochloric acid) or a hydrocarbylsulphonic acid (e.g. p-toluenesulphonic acid). According to another aspect of the invention, the reaction may be effected in water or an alcohol (e.g. isopropanol) at the reflux temperature of the solvent.

Most preferably, the reaction is effected in the presence of a hydrocarbylsulphonic acid (e.g. p-toluenesulphonic or methanesulphonic acid) or hydrochloric acid, in N-methylpyrrolidinone or dimethylacetamide at a temperature in the range 100 to 200°C, more preferably 100 to 150°C. The use of a hydrocarbylsulphonic acid (e.g. p-toluenesulophonic acid) is particularly preferred.

The compound of formula (III) is preferably used in the form of a salt, more particularly the hydrochloride salt. When the reaction is effected with the hydrochloride salt of a compound of formula (III), the addition of an acid is optional, since the hydrogen chloride associated with the compound of formula (III) provides sufficiently acidic conditions.

Compounds of formula (II) may be prepared, for example, by the method described in British patent application no. 2209335A.

Compounds of formula (III) are either known, or may be prepared from known compounds by conventional procedures.

Where a protected derivative of a compound of formula (II) is used in the above process, it may be a derivative in which the indole nitrogen atom is protected. The N-protecting group may be, for example, an arylmethoxymethyl (e.g. phenylmethoxymethyl) group. This group may be cleaved from a protected derivative of a compound of formula (I) by hydrogenolysis in the presence of a catalyst (e.g. palladium on charcoal).

Where it is desired to isolate a compound of formula (I) as a salt, for example a physiologically acceptable salt, e.g. a hydrochloride, this may be achieved by reacting the compound of formula (I) in the form of the free base with an appropriate acid, preferably with an equivalent amount, in a suitable solvent such as an alcohol (e.g. ethanol or methanol), an aqueous alcohol (e.g. aqueous ethanol), a halogenated hydrocarbon (e.g. dichloromethane), an ester (e.g. ethyl acetate), an ether (e.g. tetrahydrofuran) or a ketone (e.g. acetone). Alternatively, salt formation may take place in situ and the compound of formula (I) may be isolated directly from the reaction mixture in the form of a salt.

Physiologically acceptable salts may also be prepared from other salts, including other physiologically acceptable salts, of the compound of formula (I) using conventional methods.

Individual enantiomers of the compounds of the invention may be obtained by resolution of a mixture of enantiomers (e.g a racemic mixture) using conventional means, such as an optically active resolving acid; see for example 'Stereochemistry of Carbon Compounds' by E.L.Eliel (McGraw Hill, 1962) and 'Tables of Resolving Agents' by S. H. Wilen.

According to a preferred embodiment, the process of the invention may be used for the preparation of compounds of formula (I) in which R¹ represents a hydrogen atom or a C₁₋₃alkyl (e.g. methyl, ethyl, n-propyl or isopropyl) group, R² and R³ represent hydrogen atoms, R⁴ represents a methyl group and n represents 2.

More particularly the process of the present invention may be used to prepare 2,3,4,5-tetrahydro-5-methyl-2-[(5-methyl-1H-imidazol-4-yl)methyl]-1H-pyrido[4,3-b]indol-1-one and its physiologically acceptable salts (e.g. hydrochloride) and solvates.

The invention is illustrated by the following Examples which all describe the preparation of 2,3,4,5-tetrahydro-5-methyl-2-[(5-methyl-1H-imidazol-4-yl)methyl]-1H-pyrido[4,3-b]indol-1-one (Compound X). All temperatures are in °C. Thin layer chromatography (t.l.c.) was carried out on silica. Solvent System A as used for t.l.c. denotes dichloromethane: ethanol : 0.88 ammonia solution.
¹H-N.m.r. spectra were obtained at 250MHz for dilute solutions in d₆-dimethyl sulphoxide. Intermediate 1 denotes 2,3,4,5-tetrahydro-5-methyl-1H-pyrido[4,3-b]indol-1-one and Intermediate 2 denotes 4-hydroxymethyl-5-methylimidazole hydrochloride.

### Example 1

A mixture of Intermediate 1 (49.97g), p-toluenesulphonic acid monohydrate (9.50g) and Intermediate 2 (20.25g) in N-methylpyrrolidinone (250mℓ) was stirred and heated to 125° (over 1h) The reaction was then heated at 125-130° for 4.5h, during which time two further portions of Intermediate 2 (17.51g and 6.88g) were added. The reaction mixture was cooled, diluted with water (100mℓ), and the stirred mixture was treated slowly with 8% aqueous sodium bicarbonate (750mℓ). The resultant suspension was stirred in an ice bath for 1h and then filtered to give a solid (57.64g). A portion of this solid (11.09g) was dissolved in dichloromethane (307mℓ) and ethanol (166mℓ), boiled with decolourising charcoal for 10min and then filtered. The dichloromethane was distilled off at atmospheric pressure until the temperature of the mixture was at 65°. The stirred mixture was cooled and the resulting precipitate was filtered off to give Compound X (9.28g), t.l.c. (System A, 50:8:1) Rf 0.55 .
¹H-n.m.r:
2.20(3H,s), 3.03(2H,t), 3.64(2H,m), 3.71(3H,s), 4.50(2H,s), 7.19(2H,m), 7.44(1H,s), 7.50(1H,d), 7.99(1H,d), 11.76(1H,s).

### Example 2

Hydrogen chloride gas (1g) was bubbled into N-methylpyrrolidinone (10ml). To this solution was added Intermediate 1 (2g) and Intermediate 2 (0.74g), and the solution was heated to ca. 130° under nitrogen. After 30min, a further portion of Intermediate 2 (0.74g) was added and heating was continued for a further 4h. The solution was allowed to cool, added to water (30ml) and 1M sodium bicarbonate solution (40ml) added to pH 7-8. After standing for 2h, the precipitated solid was filtered off, washed with water (2x10ml) and dried in vacuo at 40° to give Compound X (1.3g),t.l.c. (System A, 50:8:1) Rf 0.59. The ¹H-n.m.r. data for this material were consistent with those obtained for the product of Example 1.

### Example 3

A mixture of Intermediate 1 (2.00g), Intermediate 2 (2.97g) and p-toluenesulphonic acid monohydrate (0.48g) in xylene (24ml) was stirred and heated to reflux over 0.75h. The reaction mixture was heated at reflux, with stirring, for a further 4h, then cooled to ambient temperature. The solvent was decanted and the residual semi-solid was triturated with xylene (20ml). The xylene was decanted and the residue was dissolved in water (20ml). The solution was treated with 2N sodium hydroxide (to pH 14). A gum was deposited which was triturated with water (20ml) and ethanol (15ml) to give Compound X (1.30g). The t.l.c. data for this material were consistent with those obtained for the product of Example 1.

### Example 4

A mixture of Intermediate 1 (1.50g) and Intermediate 2 (1.89g) in glacial acetic acid (10ml) was stirred and heated to reflux over 0.75h, then heated at reflux for 5.25h, during which time a further portion of Intermediate 2 (1.63g) was added. The reaction mixture was cooled to 40° and basified (to pH 14) with 5N sodium hydroxide (35ml). The mixture was saturated with solid potassium carbonate and extracted with a mixture of ethyl acetate and ethanol (1:1; 100ml). Evaporation of the extracts gave a gum which was purified by FCC eluting with ethyl acetate/methanol (4:1) to give Compound X (0.70g). The ¹H-n.m.r. and t.l.c. data for this material were consistent with those obtained for the product of Example 1.

### Example 5

A mixture of Intermediate 1 (1.50g), Intermediate 2 (1.89g) and p-toluenesulphonic acid monohydrate (0.29g) in 1-butanol (10ml) was stirred and heated to reflux over 1h, then heated at reflux for a further 23h, during which time a further portion of Intermediate 2 (1.89g) was added. The reaction mixture was cooled to ambient temperature, treated with water (10ml) and then with 8% aqueous sodium bicarbonate (20ml). The mixture was cooled to 5° and treated with ethyl acetate (25ml). The resulting suspension was filtered and the residue was washed with water to give Compound X (1.51g). The ¹H-n.m.r. and t.l.c. data for this material were consistent with those obtained for the product of Example 1.

### Example 6

A mixture of Intermediate 1 (1.50g), Intermediate 2 (2.50g) and p-toluenesulphonic acid monohydrate (0.38g) in dimethylacetamide (10ml) was stirred and heated to 125° over 0.5h, then heated at 125° for a further 3.75h, during which time a further portion of Intermediate 2 (0.50g) was added. The reaction mixture was cooled to ambient temperature, treated with water (10ml) and then, dropwise with stirring, with 8% aqueous sodium bicarbonate (20ml). The resultant suspension was cooled to 5° and filtered to give a solid which was washed with water to give Compound X (1.54g). The ¹H-n.m.r. and t.l.c. data for this material were consistent with those obtained for the product of Example 1.

### Example 7

A mixture of Intermediate 1 (1.50g), Intermediate 2 (2.08g) and concentrated hydrochloric acid (0.4ml) in 1-methyl-2-pyrrolidinone (10ml) was stirred and heated to 115° over 0.5h, then heated at 115-120° for 3.5h. The reaction mixture was cooled to ambient temperature, treated with water (10ml) and then with 8% aqueous sodium bicarbonate (28ml). The resultant suspension was cooled to 5° and filtered to give a solid which was washed with water to give Compound X (1.58g). The ¹H-n.m.r. and t.l.c. data for this material were consistent with those obtained for the product of Example 1.

### Example 8

A mixture of Intermediate 1 (1.50g), Intermediate 2 (1.89g) and methanesulphonic acid (0.19g) in 1-methyl-2-pyrrolidinone (10ml) was stirred and heated to 123° over 0.8h, then heated at 117-124° for 1h. The reaction mixture was cooled to ambient temperature, treated with water (6ml) and then with 8% aqueous sodium bicarbonate (20ml), dropwise with stirring. The resultant suspension was cooled to 2° and filtered to give a solid which was washed with water to give Compound X (1.47g). The ¹H-n.m.r. and t.l.c. data for this material were consistent with those obtained for the product of Example 1.

### Example 9

A mixture of Intermediate 1 (1.50g), Intermediate 2 (1.89g) and maleic acid (0.20g) in 1-methyl-2-pyrrolidinone (10ml) was stirred and heated to 124° over 0.75h, then heated at 112-125° for 2.25h. The reaction mixture was cooled to ambient temperature, treated with water (6ml) and then with 8% aqueous sodium bicarbonate (22ml), dropwise with stirring. The resultant suspension was cooled to 5° and filtered to give a solid which was washed with water and ethanol to give Compound X (1.03g). The ¹H-n.m.r. and t.l.c. data for this material were consistent with those obtained for the product of Example 1.

### Example 10

A mixture of Intermediate 1 (1.50g) and Intermediate 2 (1.89g) in 1-methyl-2-pyrrolidinone (10ml) was stirred and heated to 113° over 0.5h, then heated at 113-126° for 1.2h. The reaction mixture was cooled to ambient temperature and treated with water (6ml), followed by 8% aqueous sodium bicarbonate (20ml), dropwise with stirring. The resultant suspension was cooled to 5° and filtered to give a solid which was washed with water and ethanol to give Compound X (1.05g). The ¹H-n.m.r. and t.l.c. data for this material were consistent with those obtained for the product of Example 1.

### Example 11

A mixture of Intermediate 1 (10.0g), Intermediate 1 (13.4g) and p-toluenesulphonic acid monohydrate (2.38g) in 1-methyl-2-pyrrolidinone (40mℓ) was stirred and heated to 128° over 0.75h, then heated at 122-140° for a further 1.1h. The reaction mixture was then cooled to 5° and filtered to give a solid which was washed with ethanol to give Compound X in the form of its hydrochloride salt (9.95g), m.p. 281-282° (dec.). The t.l.c. data for this material were consistent with those obtained for the product of Example 1.

## Claims

1. A process for the preparation of a compound of general formula (I): 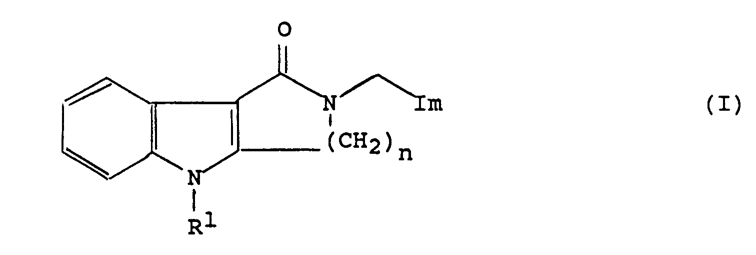 wherein Im represents an imidazolyl group of the formula : 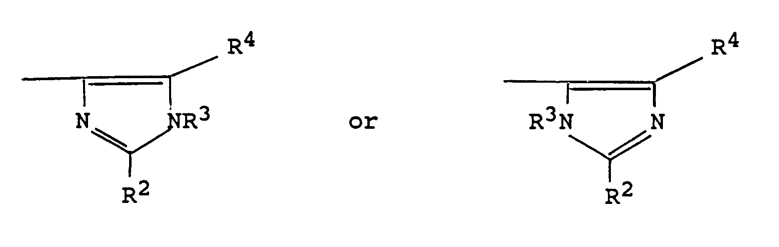 and R¹ represents a hydrogen atom or a group selected from C₁₋₆alkyl, C₃₋₆alkenyl, C₃₋₁₀alkynyl, C₃₋₇cycloalkyl, C₃₋₇cycloalkylC₁₋₄alkyl, phenyl, phenylC₁₋₃alkyl, phenylmethoxymethyl, phenoxyethyl, phenoxymethyl, -CO₂R₅, -COR⁵, -CONR⁵R⁶ or -SO₂R⁵ (wherein R⁵ and R⁶, which may be the same or different, each represents a hydrogen atom, a C₁₋₆alkyl or C₃₋₇cycloalkyl group, or a phenyl or phenylC₁₋₄alkyl group, in which the phenyl group is optionally substituted by one or more C₁₋₄alkyl, C₁₋₄alkoxy or hydroxy groups or halogen atoms, with the proviso that R⁵ does not represent a hydrogen atom when R¹ represents a group -CO₂R⁵ or -SO₂R⁵);
one of the groups represented by R², R³ and R⁴ is a hydrogen atom or a C₁₋₆alkyl, C₃₋₇cycloalkyl, C₃₋₆alkenyl, phenyl or phenylC₁₋₃alkyl group, and each of the other two groups, which may be the same or different, represents a hydrogen atom or a C₁₋₆alkyl group; and n represents 2 or 3;
which comprises reacting a compound of formula (II) 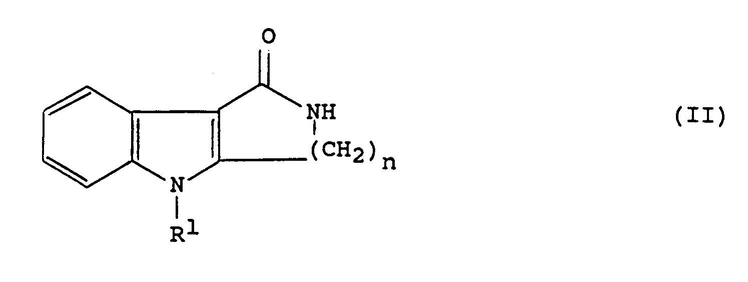 or a protected derivative thereof, with a compound of formula (III):
HOCH₂-Im (III)
or a salt thereof in the presence of an acid at an elevated temperature, followed where necessary by removal of any protecting groups.

2. A process as claimed in Claim 1 for the preparation of 2,3,4,5-tetrahydro-5-methyl-2-[(5methyl-1H-imidazol-4-yl)methyl]-1H-pyrido[4,3-b]indol-1-one by reaction of 2,3,4,5-tetrahydro-5-methyl-1H-pyrido[4,3-b]indol-1-one as the compound of formula (II) and 4-hydroxymethyl-5-methylimidazole as the compound of formula (III), the compound of formula (III) optionally being used in the form of the hydrochloride salt.

3. A process as claimed in Claim 1 or 2 wherein the acid is a strong mineral acid or a hydrocarbylsulphonic acid.

4. A process as claimed in any of Claims 1 to 3 wherein the acid is p-toluenesulphonic acid.

5. A process as claimed in any of Claims 1 to 4 wherein the reaction is carried out in a high boiling polar solvent.

6. A process as claimed in any of Claims 1 to 5 wherein the reaction is carried out in N-methylpyrrolidinone or dimethylacetamide.

7. A process as claimed in Claim 5 or 6 wherein the reaction is carried out at a temperature of 100 to 200°C.

8. A process as claimed in any of Claims 1 to 4 wherein the reaction is carried out in water or an alcohol at the reflux temperature of the solvent.

9. A process as claimed in any of Claims 1 to 8 wherein the compound of formula (I) produced is subsequently converted into a salt.

10. A process as claimed in Claim 9 wherein the compound of formula (I) is converted into the hyrdochloride.

11. A process as claimed in Claim 1 for the preparation of a compound of formula (I) in which R¹ represents a hydrogen atom or a methyl, ethyl, n-propyl or isopropyl group, R² and R³ each represent a hydrogen atom, R⁴ represents a methyl group, and n represents 2, or a physiologically acceptable salt or solvate thereof.

## Patentansprüche

1. Verfahren zur Herstellung einer Verbindung der allgemeinen Formel (I): 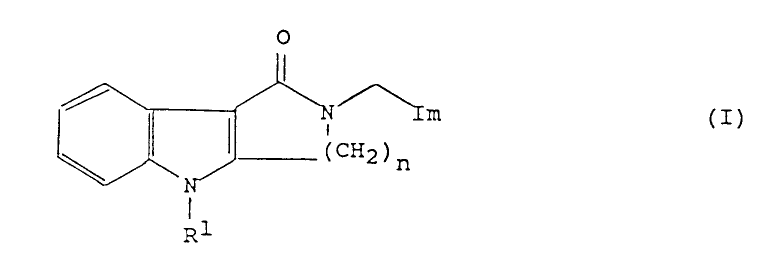 worin
Im für eine Imidazolylgruppe der Formel: 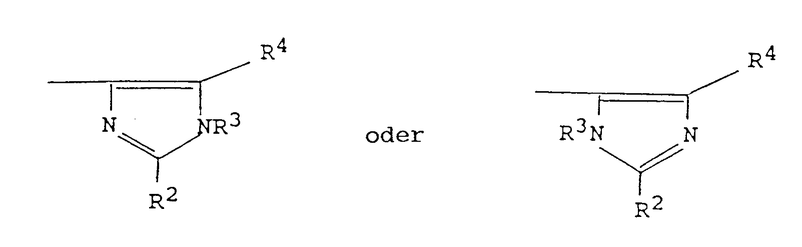 steht; und
R¹ ein Wasserstoffatom oder eine Gruppe, ausgewählt aus C₁₋₆-Alkyl, C₃₋₆-Alkenyl, C₃₋₁₀-Alkinyl, C₃₋₇-Cycloalkyl, C₃₋₇-Cycloalkyl-C₁₋₄-alkyl, Phenyl, Phenyl-C₁₋₃-alkyl, Phenylmethoxymethyl, Phenoxyethyl, Phenoxymethyl, -CO₂R⁵, -COR⁵, -CONR⁵R⁶ oder -SO₂R⁵ (wobei R⁵ und R⁶, die gleich oder verschieden sein können, jeweils ein Wasserstoffatom, eine C₁₋₆-Alkyl- oder C₃₋₇-Cycloalkylgruppe oder eine Phenyl- oder Phenyl-C₁₋₄-alkylgruppe bedeuten, wobei die Phenylgruppe gegebenenfalls durch ein oder mehrere C₁₋₄-Alkyl-, C₁₋₄-Alkoxy- oder Hydroxygruppen oder Halogenatome substituiert ist, mit der Maßgabe, daß R⁵ kein Wasserstoffatom darstellt, wenn R¹ eine Gruppe -CO₂R⁵ oder -SO₂R⁵ ist) ist;
eine der Gruppen R², R³ und R⁴ ein Wasserstoffatom oder eine C₁₋₆-Alkyl-, C₃₋₇-Cycloalkyl-, C₃₋₆-Alkenyl-, Phenyl- oder Phenyl-C₁₋₃-alkylgruppe darstellt und jede der anderen zwei Gruppen, die gleich oder verschieden sein können, jeweils ein Wasserstoffatom oder eine C₁₋₆-Alkylgruppe darstellt; und n den Wert 2 oder 3 hat; dadurch **gekennzeichnet,** daß man eine Verbindung der Formel (II): 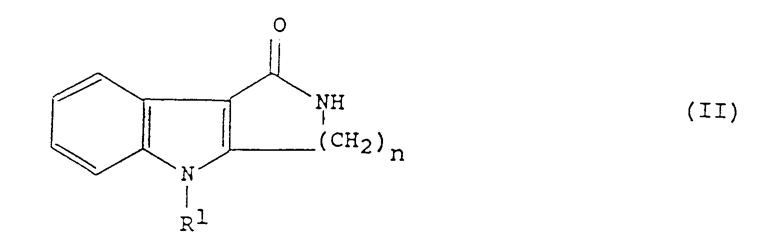 oder ein geschütztes Derivat davon mit einer Verbindung der Formel (III):
HOCH₂-Im (III)
oder einem Salz davon in Gegenwart einer Säure bei erhöhter Temperatur umsetzt und daß man erforderlichenfalls anschließend irgendwelche Schutzgruppen entfernt.

2. Verfahren nach Anspruch 1 zur Herstellung von 2,3,4,5-Tetrahydro-5-methyl-2-[(5-methyl-1H-imidazol-4-yl)methyl]-1H-pyrido[4,3-b]indol-1-on durch Umsetzung von 2,3,4,5-Tetrahydro-5-methyl-1H-pyrido[4,3-b]indol-1-on als Verbindung der Formel (II) und 4-Hydroxymethyl-5-methylimidazol als Verbindung der Formel (III), wobei die Verbindung der Formel (III) gegebenenfalls in der Form des Hydrochloridsalzes verwendet wird.

3. Verfahren nach Anspruch 1 oder 2, dadurch **gekennzeichnet,** daß die Säure eine starke Mineralsäure oder eine Hydrocarbylsulfonsäure ist.

4. Verfahren nach einem der Ansprüche 1 bis 3, dadurch **gekennzeichnet,** daß die Säure p-Toluolsulfonsäure ist.

5. Verfahren nach einem der Ansprüche 1 bis 4, dadurch **gekennzeichnet,** daß man die Reaktion in einem hochsiedenden polaren Lösungsmittel durchführt.

6. Verfahren nach einem der Ansprüche 1 bis 5, dadurch **gekennzeichnet,** daß man die Reaktion in N-Methylpyrrolidinon oder Dimethylacetamid durchführt.

7. Verfahren nach Anspruch 5 oder 6, dadurch **gekennzeichnet,** daß man die Reaktion bei einer Temperatur von 100 bis 200°C durchführt.

8. Verfahren nach einem der Ansprüche 1 bis 4, dadurch **gekennzeichnet,** daß man die Reaktion in Wasser oder einem Alkohol bei Rückflußtemperatur des Lösungsmittels durchführt.

9. Verfahren nach einem der Ansprüche 1 bis 8, dadurch **gekennzeichnet,** daß man die hergestellte Verbindung der Formel (I) danach in ein Salz umwandelt.

10. Verfahren nach Anspruch 9, dadurch **gekennzeichnet,** daß man die Verbindung der Formel (I) in das Hydrochlorid umwandelt.

11. Verfahren nach Anspruch 1 zur Herstellung einer Verbindung der Formel (I), worin R¹ ein Wasserstoffatom oder eine Methyl-, Ethyl-, n-Propyl- oder Isopropylgruppe darstellt, R² und R³ jeweils für ein Wasserstoffatom stehen, R⁴ eine Methylgruppe darstellt und n den Wert 2 hat, oder eines physiologisch annehmbaren Salzes oder Solvats davon.

## Revendications

1. Procédé de préparation d'un composé de formule générale (I) : 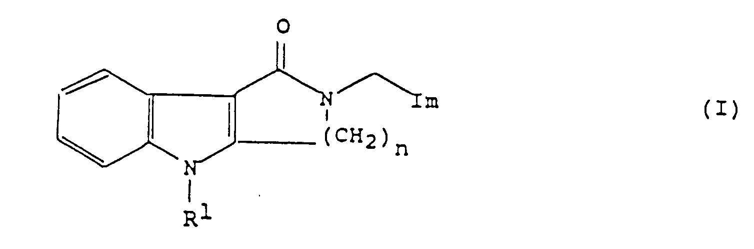 dans laquelle Im représente un groupe imidazolyle de formule : 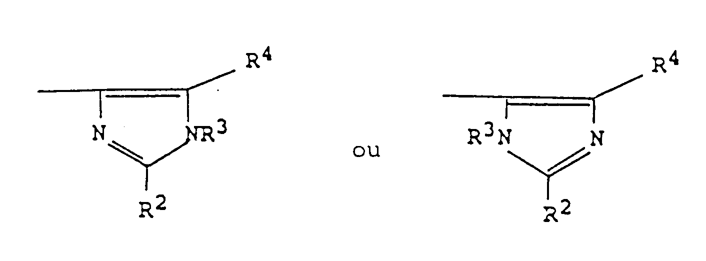 et R¹ représente un atome d'hydrogène ou un groupe choisi parmi un groupe alkyle en C₁-C₆, alkényle en C₃-C₆, alkynyle en C₃-C₁₀, cycloalkyle en C₃-C₇, (cycloalkyl en C₃-C₇) -(alkyle en C₁-C₄), phényle, phényl(alkyle en C₁-C₃), phénylméthoxyméthyle, phénoxyéthyle, phénoxyméthyle, -CO₂R⁵, -COR⁵, -CONR⁵R⁶ ou -SO₂R⁵ (dans lesquels R⁵ et R⁶ qui peuvent être identiques ou différents, représentent chacun un atome d'hydrogène, un groupe alkyle en C₁-C₆ ou cycloakyle en C₃-C₇, ou un groupe phényle ou phényl(alkyl en C₁-C₄), le groupe phényle étant éventuellement substitué avec un ou plusieurs groupes alkyle en C₁-C₄, alkoxy en C₁-C₄, hydroxy, ou un ou plusieurs atomes d'halogène, à condition que R⁵ ne représente pas un atome d'hydrogène lorsque R¹ représente un groupe -CO₂R⁵ ou -SO₂R⁵) ;
l'un des groupes représentés par R², R³ et R⁴, est un atome d'hydrogène ou un groupe alkyle en C₁₋C₆, cycloalkyle en C₃-C₇, alkényle en C₃-C₆, phényle ou phényl(alkyle en C₁-C₃), et chacun des deux autres groupes qui peuvent être identiques ou différents, représente un atome d'hydrogène ou un groupe alkyle en C₁-C₆ ; et n est égal à 2 ou 3 ;
selon lequel on fait réagir un composé de formule (II) : 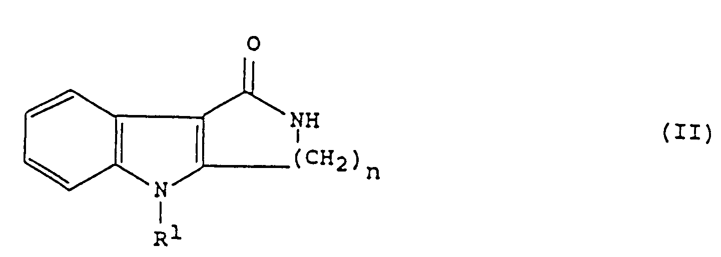 ou un dérivé protégé de celui-ci, avec un composé de formule (III) :
HOCH₂-Im (III)
ou un sel de celui-ci, en présence d'un acide à température élevée, puis lorsque cela est nécessaire, on élimine tout groupe protecteur.

2. Procédé selon la revendication 1, pour la préparation de la 2,3,4,5-tétrahydro-5-méthyl-2-[(5-méthyl-1H-imidazol-4-yl)méthyl-1H-pyrido[4,3-b]indol-1-one, en faisant réagir la 2,3,4,5-tétrahydro-5-méthyl-1H-pyrido[4,3-b]indol-1-one en tant que le composé de formule (II) et du 4-hydroxyméthyl-5-méthylimidazole en tant que le composé de formule (III), le composé de formule (III) étant éventuellement employé sous la forme du sel chlorhydrate.

3. Procédé selon la revendication 1 ou 2, dans lequel l'acide est un acide minéral fort ou un acide hydrocarbyl sulfonique.

4. Procédé selon l'une quelconque des revendications 1 à 3, dans lequel l'acide est l'acide p-toluène sulfonique.

5. Procédé selon l'une quelconque des revendications 1 à 4, dans lequel la réaction est effectuée dans un solvant polaire à point d'ébullition élevé.

6. Procédé selon l'une quelconque des revendications 1 à 5, dans lequel la réaction est effectuée dans de la N-méthylpyrrolidinone ou du diméthyl acétamide.

7. Procédé selon la revendication 5 ou 6, dans lequel la réaction est effectuée à une température de 100 à 200 °C.

8. Procédé selon l'une quelconque des revendications 1 à 4, dans lequel la réaction est effectuée dans l'eau, ou un alcool à la température de reflux du solvant.

9. Procédé selon l'une quelconque des revendications 1 à 8, dans lequel le composé de formule (I) produit, est ensuite converti en un sel.

10. Procédé selon la revendication 9, dans lequel le composé de formule (I) est converti en le chlorhydrate.

11. Procédé selon la revendication 1, pour la préparation de composé de formule (I) dans laquelle R¹ représente un atome d'hydrogène, ou un groupe méthyle, éthyle, N-propyle ou isopropyle, R² et R³ représentent chacun un atome d'hydrogène, R⁴ représente un groupe méthyle et n est égal à 2, ou d'un sel physiologiquement acceptable ou d'un solvat de celui-ci.
